# EUROPEAN PATENT SPECIFICATION

(11) **EP 4 138 905 B1**
(45) Date of publication and mention of the grant of the patent: **14.10.2026**
(21) Application number: 21792923.1
(22) Date of filing: 20.04.2021
(51) Int. Cl.: A61K 38/39, A61P 31/14

(54) **USE OF SURFACTANT PROTEIN D TO TREAT INFECTION BY SARS-COV-2 VARIANTS**
VERWENDUNG VON SURFACTANT-PROTEIN D ZUR BEHANDLUNG VON INFEKTIONEN DURCH SARS-COV-2-VARIANTEN
UTILISATION DE LA PROTÉINE TENSIOACTIVE D (SP-D) POUR TRAITER L'INFECTION PAR UN VARIANT DU SARS-COV-2

(30) Priority: 22.04.2020 US 202063013726 P; 31.08.2020 US 202063072354 P
(43) Date of publication of application: 01.03.2023
(73) Proprietor: Airway Therapeutics, Inc., Marietta, GA 30068 (US)
(72) Inventor: KINGMA, Paul, Marietta, Georgia 30068 (US); GRANT, Shawn, Marietta, Georgia 30068 (US); ARROYO-RODRÍGUEZ, Raquel, Marietta, Georgia 30068 (US); SALZBERG, Marc, Marietta, Georgia 30068 (US)
(74) Representative: Zacco Sweden AB
(86) International application number: PCT/US2021/028207
(87) International publication number: WO 2021/216584

(56) References cited:
- WO-A1-2020/247675
- WO-A1-2021/191592
- CN-A- 112 481 417
- KR-B1- 102 205 028
- US-A1- 2003 221 199
- US-A1- 2011 189 104
- US-A1- 2016 333 074
- ANONYMOUS: "Airway Therapeutics Announces Filing with NIH to Evaluate AT-100 as a Therapy for Novel Coronavirus | Airway Therapeutics", 11 March 2020 (2020-03-11), XP093104997, Retrieved from the Internet <URL:https://www.airwaytherapeutics.com/airway-therapeutics-announces-filing-with-nih-to-evaluate-at-100-as-a-therapy-for-novel-coronavirus/> [retrieved on 20231123]
- TAREK MOHAMED ABD EL-AZIZ ET AL: "Recent progress and challenges in drug development against COVID-19 coronavirus (SARS-CoV-2) - an update on the status", INFECTION , GENETICS AND EVOLUTION, vol. 83, 19 April 2020 (2020-04-19), NL, pages 104327, XP055693374, ISSN: 1567-1348, DOI: 10.1016/j.meegid.2020.104327
- LETH-LARSEN ET AL: "The SARS coronavirus spike glycoprotein is selectively recognized by lung surfactant protein D and activates macrophages", IMMUNOBIOLOGY, URBAN UND FISCHER VERLAG, DE, vol. 212, no. 3, 3 April 2007 (2007-04-03), pages 201 - 211, XP022015156, ISSN: 0171-2985, DOI: 10.1016/J.IMBIO.2006.12.001
- MADAN TARUNA ET AL: "A Recombinant Fragment of Human Surfactant Protein D Binds Spike Protein and Inhibits Infectivity and Replication of SARS-CoV-2 in Clinical Samples", AMERICAN JOURNAL OF RESPIRATORY CELL AND MOLECULAR BIOLOGY., vol. 65, no. 1, 30 March 2021 (2021-03-30), NEW YORK, NY, US, pages 41 - 53, XP093023051, ISSN: 1044-1549, DOI: 10.1165/rcmb.2021-0005OC
- GRANT OLIVER C., MONTGOMERY DAVID, ITO KEIGO, WOODS ROBERT J.: "Analysis of the SARS-CoV-2 spike protein glycan shield: implications for immune recognition", BIORXIV, 1 May 2020 (2020-05-01), pages 1 - 17, XP055868020, DOI: 10.1101/2020.04.07.030445

## Description

### REFERENCE TO SEQUENCE LISTING

The present application is being filed along with a Sequence Listing in electronic format. The Sequence Listing is provided as a file entitled AIRWY017SEQLIST, created April 14, 2021, which is approximately 6 Kb in size.

### FIELD OF THE INVENTION

A pharmaceutical composition is provided herein for use in treating or ameliorating a viral infection comprising a coronavirus in a subject, wherein the pharmaceutical composition comprises recombinant human surfactant protein D (rhSP-D). The viral infection comprises severe acute respiratory syndrome coronavirus 2 (SARS-CoV-2), wherein the SARS-CoV-2 comprises an S1 protein comprising an N501Y mutation or a D614G mutation.

### BACKGROUND OF THE INVENTION

A new human disease, coronavirus disease 2019 (COVID-19) caused by severe acute respiratory syndrome coronavirus 2 (SARS-CoV-2) has emerged. SARS-CoV-2 belongs to a family of coronaviruses which also includes severe acute respiratory syndrome coronavirus (SARS-CoV-1) and Middle East respiratory syndrome-related coronavirus (MERS-CoV), which cause severe acute respiratory syndrome (SARS) and Middle East respiratory syndrome (MERS), respectively.

COVID-19 was first identified in December 2019 in Wuhan, the capital of China's Hubei province, and has since spread globally, resulting in a coronavirus pandemic. Common symptoms include fever, cough, and shortness of breath. Other symptoms may include fatigue, muscle pain, diarrhea, sore throat, loss of smell, and abdominal pain. While the majority of patients result in mild symptoms, some cases progress to viral pneumonia and multi-organ failure. Patients are managed with supportive care, which may include fluid therapy, oxygen support, and supporting other affected vital organs. There is a need for treatments for COVID-19 and related viral disorders.

US Patent Application Publication No. 20110189104A1 provides that surfactant protein D (SP-D) is a 43-kDa member of the collectin family of collagenous lectin domain-containing proteins that is expressed in epithelial cells of the lung. It describes methods and compositions for the treatment of disorders associated with lung injury, including methods and compositions for the treatment of bronchopulmonary disorder (BPD) using recombinant human surfactant protein D and surfactant formulations.

The publication "Analysis of the SARS-CoV-2 spike protein glycan shield: implications for immune recognition," to Oliver C. Grant et al., bioRxiv 2020.04.07.030445; doi: https://doi.org/10.1101/2020.04.07.030445, describes 3D structures of glycoforms of the spike (S) glycoprotein generated from SARS-CoV-2, based on reported 3D structures and glycomics data for the protein produced in HEK293 cells. Structures for glycoforms representing those present in the nascent glycoproteins (prior to enzymatic modifications in the Golgi) are also analyzed, as well as those that are commonly observed on antigens present in other viruses.

### SUMMARY OF THE INVENTION

Described herein is an invention concerning a pharmaceutical composition for use in treating or ameliorating a viral infection comprising a coronavirus in a subject, wherein the pharmaceutical composition comprises a recombinant human surfactant protein D (rhSP-D), wherein the viral infection comprises SARS-CoV-2, and wherein the SARS-CoV-2 comprises an S1 protein comprising an N501Y mutation or a D614G mutation..

Embodiments of the invention are defined in claims 2-14.

### BRIEF DESCRIPTION OF THE DRAWINGS

FIG. 1A depicts a schematic overview of an ELISA assay to detect binding between immobilized SP-D and an S1 subunit of a spike protein of SARS-CoV-2 (S1-protein).
FIG. 1B depicts a line graph of absorbance with increasing concentration of S1-protein in an assay for binding between immobilized SP-D and the S1-protein with a first sample of immobilized SP-D in the presence of calcium, of EDTA, or of maltose, in which plates were coated using 5 µg/mL SP-D.
FIG. 1C depicts a line graph of absorbance with increasing concentration of S1-protein in an assay for binding between immobilized SP-D and the S1-protein with a second sample of immobilized SP-D in the presence of calcium, of EDTA, or of maltose, in which plates were coated using 5 µg/mL SP-D.
FIG. 1D depicts a line graph of absorbance with increasing concentration of S1-protein in an assay for binding between immobilized SP-D and the S1-protein with a first sample of immobilized SP-D in the presence of calcium, of EDTA, or of maltose, in which plates were coated using 2 µg/mL SP-D.
FIG. 1E depicts a line graph of absorbance with increasing concentration of S1-protein in an assay for binding between immobilized SP-D and the S1-protein with a second sample of immobilized SP-D in the presence of calcium, of EDTA, or of maltose, in which plates were coated using 2 µg/mL SP-D.
FIG. 2A depicts a schematic overview of an ELISA assay to detect binding between SP-D and immobilized S1-protein.
FIG. 2B depicts a graph of absorbance with increasing concentration of SP-D in an assay for binding between SP-D and immobilized S1-protein with a first sample of immobilized SP-D in the presence of calcium, or of EDTA.
FIG. 2C depicts a graph of absorbance with increasing concentration of SP-D in an assay for binding between SP-D and immobilized S1-protein with a second sample of immobilized SP-D in the presence of calcium, or of EDTA.
FIG. 3 is a graph of SP-D concentration in bronchoalveolar lavage fluid obtained from COVID-19 patients, and also in control subjects previously reported in literature. Error bars represent 1.5 times the interquartile rate (Q1 to Q3).
FIG. 4A is a graph of absorbance units for various concentrations of rhSP-D in an ELISA to measure rhSP-D binding to immobilized S1-protein of SARS-CoV-2 (Wuhan variant).
FIG. 4B is a graph of absorbance units for various concentrations of S1-protein (Wuhan variant) in an ELISA to measure SARS-CoV-2 S1-protein binding to immobilized rhSP-D.
FIG. 4C is a graph of absorbance units for various concentrations of rhSP-D in an ELISA to measure rhSP-D binding to immobilized S1-protein variants of SARS-CoV-2 (Wuhan variant; U.K. variant; and South Africa variant).
FIG. 4D is a graph of absorbance units for various concentrations of rhSP-D in an ELISA to measure rhSP-D binding to an immobilized S1-protein variant of SARS-CoV-2 containing a single mutation (N501Y).
FIG. 4E is a graph of absorbance units for various concentrations of rhSP-D in an ELISA to measure rhSP-D binding to an immobilized S1-protein variant of SARS-CoV-2 containing a single mutation (D614G).
FIG. 5A depicts a scheme for a bridge assay between S1-protein and maltose-coated beads via rhSP-D in which rhSP-D is pre-mixed with S1-protein before addition of maltose-coated beads.
FIG. 5B depicts a scheme for a bridge assay between S1-protein and maltose beads via rhSP-D in which rhSP-D is pre-incubated with maltose-coated before addition of S1-protein.
FIG. 5C depicts a SDS-PAGE gel for the scheme shown in FIG. 5A in which the gel was developed by silver-staining to detect S1-protein (migrates as 100-140 kDa) and rhSP-D (43 kDa).
FIG. 5D depicts a SDS-PAGE gel for the scheme shown in FIG. 5B in which the gel was developed by silver-staining to detect S1-protein (migrates as 100-140 kDa) and rhSP-D (43 kDa).
FIG. 5E is a bar graph for relative densitometry of eluted (P) bands from the pre-mix approach and the 1^{st}-rhSP-D, at 4 µg of rhSP-D in the presence of S1-protein or buffer. Error bars represent standard deviation, densitometry (n=2).
FIG. 6A depicts a line graph for the results of an ELISA to determine binding of ACE2 to immobilized S 1-protein in the presence of various concentrations of rhSP-D.
FIG. 6B depicts a bar chart for the results of an ELISA to determine binding of ACE-2 to immobilized S1-protein in the presence of various concentrations of rhSP-D.
FIG. 6C depicts a line graph for the results of an ELISA to determine binding of S1-protein to immobilized rhSP-D in the presence of various concentrations of ACE2.
FIG. 6D depicts a bar chart for the results of an ELISA to determine binding of S1-protein to immobilized rhSP-D in the presence of various concentrations of ACE2.
FIG. 7 depicts a graph of CCID50 (50% cell culture infectious dose) of SARS-CoV-2 at various concentrations of rhSP-D. Individual data points represent the average of three replicates.

### DETAILED DESCRIPTION

Described herein is an invention concerning a pharmaceutical composition for use in treating or ameliorating a viral infection comprising a coronavirus in a subject, wherein the pharmaceutical composition comprises a recombinant human surfactant protein D (rhSP-D), wherein the viral infection comprises SARS-CoV-2, and wherein the SARS-CoV-2 comprises an S1 protein comprising an N501Y mutation or a D614G mutation.

SP-D plays a role in innate defense against some viruses, such as influenza A virus (IAV) in the lungs (Hartshorn K.L. et al. (1994) J. Clin. Invest. 94:311-319. Multivalent lectin-mediated interactions of SP-D with IAVs result in viral aggregation, reduced epithelial infection, and enhanced IAV clearance by phagocytic cells (VanEijk, M. et al., (2019) Front Immunol. 10:2476. SP-D binds to viral hemagglutinin (HA) and in particular, mannosylated glycans on the HA in a calcium dependent manner (Hsieh I.N. et al (2018) Front Immunol. 9:1368).

Coronaviruses, including SARS-CoV-2, have four structural proteins, known as the S (spike), E (envelope), M (membrane), and N (nucleocapsid) proteins; the N protein holds the RNA genome, and the S, E, and M proteins together create the viral envelope. The spike glycoprotein (S-protein) is responsible for allowing the virus to attach to and fuse with the membrane of a host cell. Coronavirus entry into host cells is mediated by the S-protein that forms homotrimers protruding from the viral surface (Walls A.C. et al. (2020) Cell 181:281-292). S-protein includes two functional subunits responsible for binding to the host cell receptor (S1 subunit) and fusion of the viral and cellular membranes (S2 subunit). For many coronaviruses, S-protein is cleaved at the boundary between the S1 and S2 subunits, which remain non-covalently bound in the prefusion conformation. The distal S1 subunit comprises the receptor-binding domain(s) and contributes to stabilization of the prefusion state of the membrane-anchored S2 subunit that contains the fusion machinery.

The S1 subunit of the S-protein comprises a receptor binding domain that interacts with the human angiotensin-converting-enzyme-2 (ACE2) receptor in type II pneumocytes. Viral recognition of the S-protein by the ACE2 receptor leads to the internalization of the virus by the host cells, resulting in viral replication. New copies of SARS-CoV-2 are externalized to infect more cells, increasing the viral load in lungs, exacerbating the pro-inflammatory response, and extending the cellular and epithelial lung damage. These pathologic events in the lungs trigger the clinical symptoms of COVID-19: fever, cough, shortness of breath, fatigue and dyspnea in mild to moderate manifestations. In severe cases, pneumonia progresses to complex ALI/ARDS, respiratory failure, septic shock and even death. To date, vaccines for this disease are still in clinical trials. Remdesivir has shown effect by shortening the recovery time of patients 4 days, and dexamethasone has reduced the mortality of critical patients by 33%. However, treatments that specifically target the virus and the exacerbated inflammatory response with higher efficacy are still needed.

New variants of SARS-CoV-2 have emerged due to the mutation of certain amino acids in the viral sequence, some of them located in the spike protein. The B.1.1.7. (so-called U.K. variant), B.1.351 (South Africa) and P.1 (Brazil) are some of the most concerning ones due to their spread around the world and/or resulting clinical disease severity (Tegally, H., et al., (2021) Nature 592:438-443; and Voloch, C. M., et al., (2021) J Virol., doi: 10.1128/jvi.00119-21). These variants enclose different mutations but, the three of them share two common mutations in the S1-protein: N501Y and D614G (Liu, Y., et al., (2021) 'The N501Y spike substitution enhances SARS-CoV-2 transmission' bioRxiv; and Rees-Spear, C., et al., (2021) Cell Rep 34: 108890). More examples of variants are disclosed in Filipe Pereira (2021) Biochem Biophys Res Commun. 550: 8-14.

Pulmonary surfactant contains four different surfactant proteins. Two hydrophobic proteins, surfactant protein B and surfactant protein C, are involved in the reduction of surface tension at the air-water interface; while two hydrophilic proteins, surfactant protein A and SP-D, are members of the collectin family and are involved in the modulation of the host immune response and in surfactant pool recycling. SP-D is a C-type (Ca²⁺-dependent) lectin that includes four domains: a cysteine-linked N-terminal region required for the formation of intermolecular disulfide bonds; a triple-helical collagen region; an α-helical-coiled-coil trimerizing neck peptide; and a C-terminal calcium-dependent carbohydrate-recognition domain (CRD) (Crouch E. et al. (1994) J Biol Chem 269:17311-9). Monomers form trimers through folding of the collagenous region into triple helices and the assembly of a coiled-coil bundle of α-helices in the neck region. These trimers are stabilized by two disulfide bonds in the cysteine-rich N-terminal domain. The SP-D trimer has a total molecular weight of 129 kDa which includes three identical 43-kDa polypeptide chains. SP-D trimers can form higher order oligomerization states which vary by size and conformation. Higher order oligomerization states may be important for SP-D function (Hakansson K, et al., Protein Sci (2000) 9:1607-17; Crouch E. Respir Res (2000) 1:93-108; Crouch E. et al. (2006) J Biol Chem 281:18008-14). Therefore, pharmaceutical compositions of SP-D should have an appropriate oligomerization state for optimal activity including binding to carbohydrate ligands on the surface of pathogens, and achieving potent bacterial and viral agglutination effects (White M, et al., J Immunol (2008) 181:7936-43). An appropriate oligomerization state also has a role in optimal receptor recognition and receptor-mediated signal transduction for modulation of the host immune response (Yamoze M et al., J Biol Chem (2008) 283:35878-35888) as well as for maintenance of surfactant homeostasis (Zhang L et al., J Biol Chem (2001) 276:19214-19219). Deletion studies with a rat SP-D protein demonstrated that the rat cysteine-linked N-terminal region had a role in efficient viral neutralization and opsonization. *See* White M. et al., (2008) J. Immunol 181:7937-7942.

SP-D binds to glycosylated ligands on pathogens such as LPS in bacteria, hemagglutinin (HA) in influenza virus, and F-protein in respiratory syncytial virus. Binding triggers opsonization, aggregation, and direct killing of microbes, which facilitates their clearance from the lungs by phagocytic cells such as macrophages. SP-D dodecamers and higher order oligomers have shown an increased activity and potency in this anti-microbial function. In addition to roles in pathogen clearance, SP-D has also shown an anti-inflammatory effect in animal models of bacterial and viral respiratory infections as well as in lung injury induced by mechanical ventilation; in both cases, SP-D has decreased the levels of pro-inflammatory cytokines (e.g. IL-6), the neutrophilic response and NETosis, and lung tissue damage. Animal models have consistently demonstrated an association between higher levels of pulmonary SP-D and improved outcomes following viral, bacterial, or mechanical lung injury. Likewise, human studies have demonstrated lower mortality rates in ARDS patients with high levels of pulmonary SP-D. Full length recombinant hSP-D has been successfully produced in mammalian cells, showing comparable structure and activity to human native SP D. Therefore, rhSP-D could be a novel class of antiviral therapeutic for COVID-19.

Disclosed herein are studies which evidence of the importance of SP-D in COVID-19 and the potential of rhSP-D as an anti-viral molecule, such as a COVID-19 anti-viral therapy. As discussed in more detail below, levels of SP-D were found to be substantially reduced in COVID-19 patients. Administration of rhSP-D would supplement the decreased pulmonary SP-D levels that were found in lungs of COVID-19 patients. In addition, binding of rhSP-D to SARS-CoV-2 spike-protein was found to inhibit viral replication in host cells, and such binding could also lead to viral aggregation resulting in a more effective clearance of the virus by phagocytic cells.

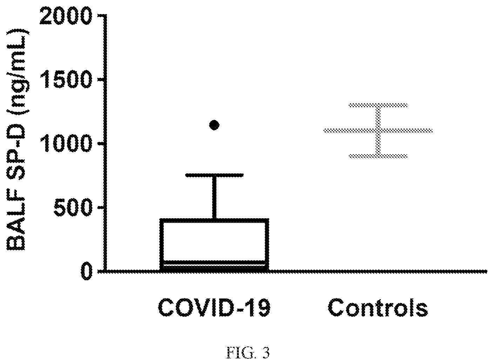

Consistent with a clinical significance of SP-D activity, a positive correlation has been shown between survival rates to ARDS and higher levels of pulmonary SP-D at the beginning of the syndrome (Greene KE, et al (1999). Am J Respir Crit Care Med 160:1843-1850). Herein, it is shown that COVID-19 patients exhibited a 3-4 times decreased concentration of pulmonary SP-D compared to non-COVID-19 control patients (FIG. 3). It was not certain if low pulmonary SP-D levels in COVID-19 patients was a result of severe SARS-CoV-2 infection or if low pulmonary SP-D levels increased a risk for developing severe COVID-19. A previous study in patients that were at risk for developing ARDS found that a lower SP-D concentration in BALF, prior to the onset of ARDS, was associated with worse outcome suggesting that the latter explanation was more likely, and that low pulmonary SP-D levels led to more severe disease. It is also uncertain if other comorbidities influence pulmonary SP-D levels in COVID-19 patients. Therefore, supplementation of COVID-19 patients with exogenous SP-D to reestablish normal and functional levels of SP-D in lungs could improve outcomes.

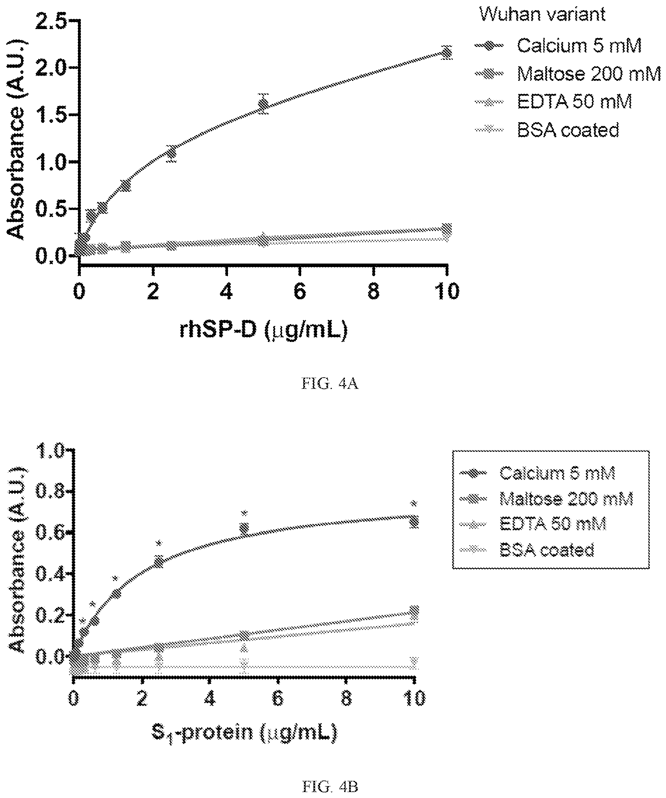

Pathogen recognition and binding to glycosylated determinants is the first step and hallmark action of SP-D to opsonize infectious agents (e.g. viruses and bacteria) and facilitate their fast clearance by phagocytic cells in the lungs, as it has been shown in *in vivo* animal models of SP-D reduction or exogenous SP-D supplementation (Wright JR. (2005) Nat Rev Immunol 2005; 5: 58-68; and Kingma PS, et al (2006) Curr Opin Pharmacol 6:277-283; LeVine AM, et al. (2004) Am J Respir Cell Mol Biol 31:193-199; Ikegami M, et al (2006) Am J Respir Crit Care Med 173:1342-1347; Hartshorn KL, et al (1998). Am J Physiol 274:L958-969; and LeVine AM, et al (2001) J Immunol 167:5868-5873). SP-D has shown calcium-dependent binding to the S-protein of the previous SARS-CoV strain and high glycosylation of the current SARS-CoV-2 S-protein has been confirmed and mapped suggesting SARS-CoV-2 S-protein may be a target of SP-D. Herein, it has been demonstrated that rhSP-D binds to the antigen of the current SARS-CoV-2 (FIG. 4A, FIG. 4B) via a process that mimics opsonization and the critical first step of clearance of SARS-CoV-2 by SP-D *in vivo.* Thus, rhSP-D could increase viral clearance and reduce viral load in COVID-19 patients.

Binding affinity of SP-D for the spike protein of the original variant from Wuhan was very similar to the variant emerged in U.K. (B.1.1.7.) which has widespread worldwide quickly. However, binding affinity to the S-protein from the South African variant (B.1.351) was significantly decreased. Many factors determine the infectivity and severity of the disease produced by the virus, recognizing that limitation, it is tempting to speculate that the decreased binding affinity of SP-D to the spike protein could be one of the factors that influence the higher virulence observed with this new South African variant, which could be translated in the virus bypassing the innate immune defense more easily. In line with this, the N501Y spike mutation enhances virus transmission. As disclosed herein, SP-D had decreased binding affinity to the spike protein with the N501Y spike mutation.

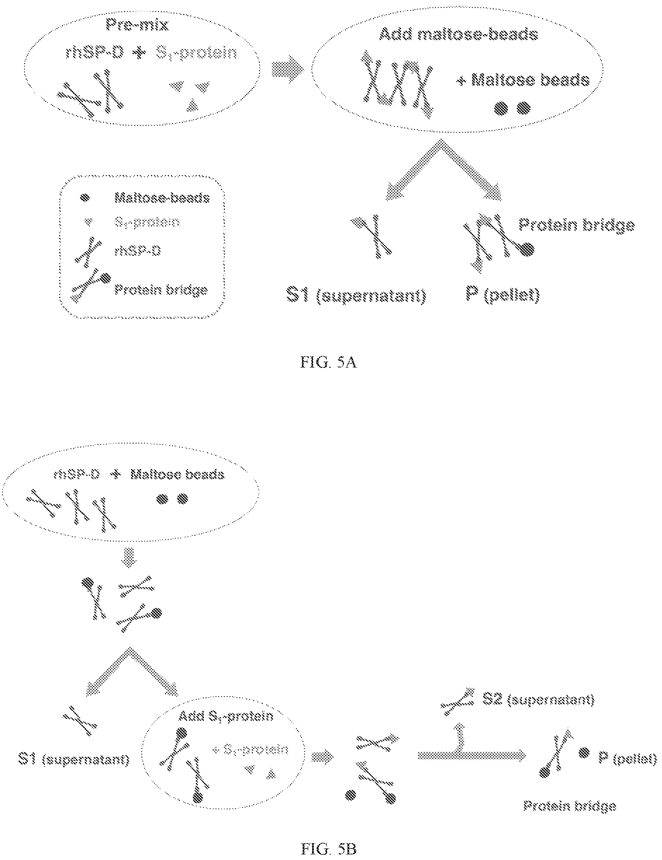

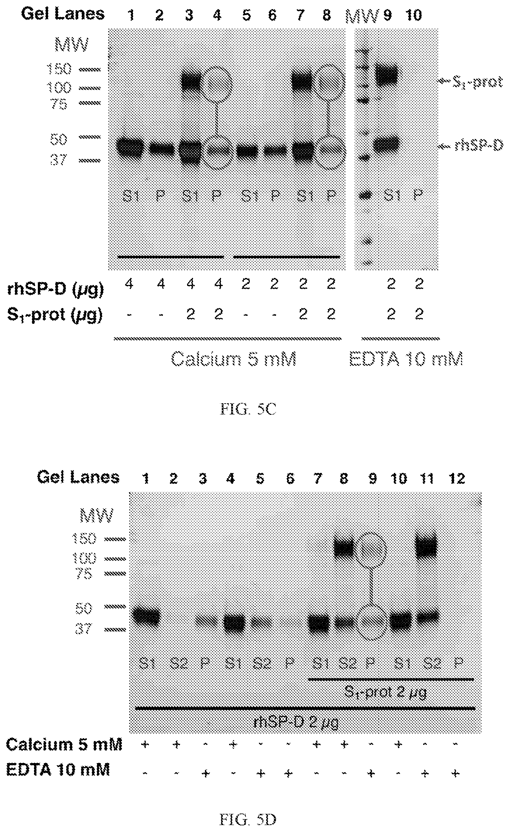

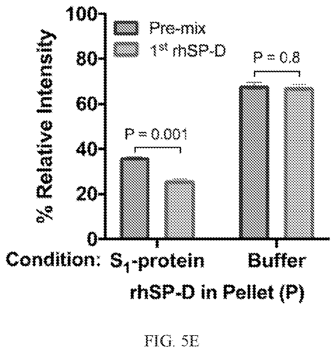

Binding of pathogens by rhSP-D leads to their aggregation, forming clusters where multiple viral molecules that are removed at once by phagocytic cells, thus making viral clearance more effective. The critical first step of aggregation is driven by the ability of SP D (hexamers, dodecamers or higher order multimers to bind more than one virus and form a protein bridge linking multiple pathogens. As disclosed herein, SP-D was able to form protein bridges between S-proteins (FIG. 5A, FIG. 5B, FIG. 5C, FIG. 5D, FIG. 5E). Studies disclosed herein demonstrated a first step of viral aggregation (i.e. binding) and the subsequent formation of the rhSP-D protein bridge. Moreover, it is likely that the presence of multiple spike-proteins on the surface of the intact virus will further facilitate viral aggregation and clearance.

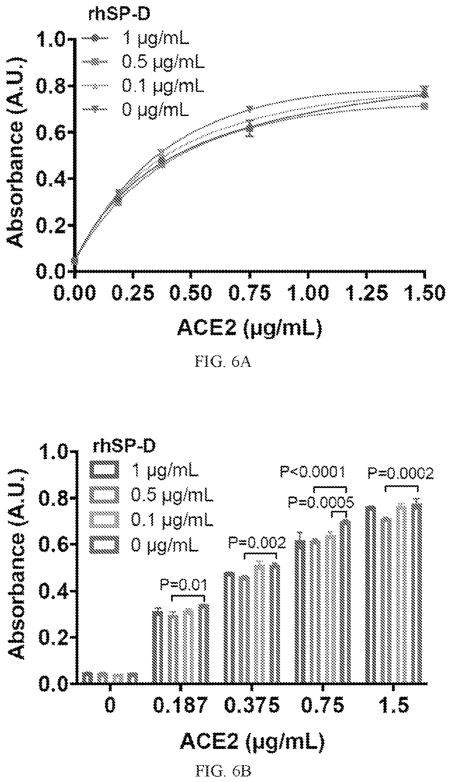

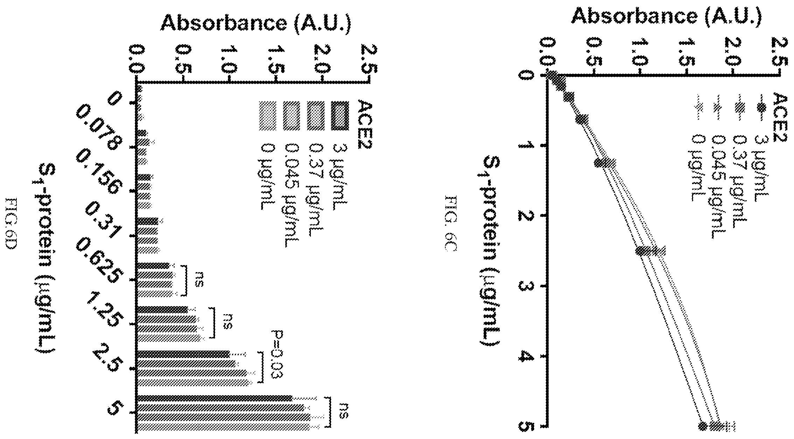

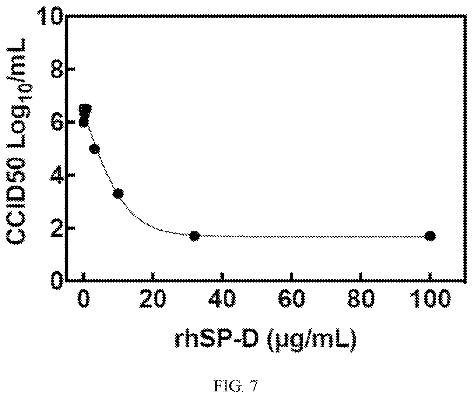

As disclosed herein, rhSP-D inhibited SARS-CoV-2 life cycle by inhibiting virus replication in cells with an EC₉₀ of 3.7 µg/mL (FIG. 7). Without wishing to be bound to any one theory, a first mechanism for rhSP-D inhibition of virus replication may include a steric blockage on the interaction between the receptor binding domain within S-protein and ACE2 by the rhSP-D bound to the glycosylated S-protein, which could restrict the accessibility of key domains in the presence of the bound SP-D molecule. However, this effect was not evident when experiments were performed with isolated S1-protein, ACE2 and rhSP-D (FIG. 6A, FIG. 6B, FIG. 6C, FIG. 6D). It is possible that steric blockage may still be observed when the conformation and position of the S-protein and ACE2 receptor are restrained on a virus envelope or cell membrane, respectively. A second mechanism for rhSP-D inhibition of virus replication may include, potential aggregation of SARS-CoV-2 induced by rhSP-D by reducing the number of viral molecules available to interact with the host cell. The first and second mechanisms may not be mutually exclusive, and may be cooperative with one another.

As disclosed herein, COVID-19 patients had reduced pulmonary levels of SP-D. Recombinant hSP-D bound the SARS-CoV-2 S-protein from different virus variants and inhibited the life cycle of the virus by inhibiting viral replication. SP-D formed protein bridges with S-protein, which would correspond to a step of viral aggregation that would enhance viral clearance from the lungs by phagocytic cells. In addition, SP-D has previously demonstrated anti-inflammatory and lung protective role in several viral and bacterial infections. SP-D has a strong potential to be a novel class of antiviral therapy that will target multiple stages of the SARS-CoV-2 infection.

Some embodiments of the compositions provided herein include aspects disclosed in U.S. Pat. No. 10975389, U.S. Pat. No. 10752914, U.S. Pat. No. 9492503, U.S. Pat. No. 6838428, U.S. 2021/0010988, and WO 2019/191247.

### Pharmaceutical compositions for use

The invention relates to a pharmaceutical composition for use in treating or ameliorating a viral infection comprising a coronavirus in a subject, wherein the pharmaceutical composition comprises recombinant human surfactant protein D (rhSP-D). According to the invention, the viral infection comprises severe acute respiratory syndrome coronavirus 2 (SARS-CoV-2). According to the invention, the SARS-CoV-2 comprises an S1 protein comprising an N501Y mutation or a D614G mutation.

In some embodiments, the pharmaceutical composition comprises a buffer, a sugar, and a calcium salt.

In some embodiments, the buffer is selected from the group consisting of acetate, citrate, glutamate, histidine, succinate, and phosphate. In some embodiments, the buffer is histidine. In some embodiments, the concentration of the histidine is from about 1 mM to about 10 mM.

In some embodiments, the sugar is selected from the group consisting of sucrose, maltose, lactose, glucose, fructose, galactose, mannose, arabinose, xylose, ribose, rhamnose, trehalose, sorbose, melezitose, raffinose, thioglucose, thiomannose, thiofructose, octa-O-acetyl-thiotrehalose, thiosucrose, and thiomaltose. In some embodiments, the sugar is lactose. In some embodiments, the concentration of the lactose is from 200 mM to 300 mM. In some embodiments, the concentration of the lactose is about 265 mM.

In some embodiments, the calcium salt is selected from the group consisting calcium chloride, calcium bromide, calcium acetate, calcium sulfate, and calcium citrate. In some embodiments, the calcium salt is calcium chloride. In some embodiments, the concentration of the calcium chloride is from about 1 mM to about 10 mM. In some embodiments, the concentration of the calcium chloride is about 5 mM.

In some embodiments, the pharmaceutical composition has a pH from about 5.0 to about 7.0. In some embodiments, the pharmaceutical composition has a pH about 6.0.

In some embodiments, the concentration of the rhSP-D is from about 0.1 mg/ml to about 10 mg/ml.

In some embodiments, the pharmaceutical composition comprises a population of rhSP-D polypeptides having oligomeric forms, wherein greater than 30% of the oligomeric forms comprise dodecamers of rhSP-D. In some embodiments, greater than 35% of the oligomeric forms comprise dodecamers of rhSP-D. In some embodiments, greater than 40% of the oligomeric forms comprise dodecamers of the rhSP-D.

In some embodiments, the pharmaceutical composition comprises a bulking agent. In some embodiments, the bulking agent is selected from the group consisting of mannitol, xylitol, sorbitol, maltitol, lactitol, glycerol, erythritol, arabitol, glycine, alanine, threonine, valine, and phenylalanine.

In some embodiments, the pharmaceutical composition lacks a chelating agent. In some embodiments, the chelating agent is selected from EDTA and EGTA.

In some embodiments, the rhSP-D comprises an amino acid sequence having at least 95% identity to the amino acid sequence of SEQ ID NO:02.

In some embodiments, the subject is mammalian. In some embodiments, the subject is human.

### Pharmaceutical compositions

The composition provided herein includes recombinant human surfactant protein D (rhSP-D). In some embodiments, rhSP-D has activity in a bacterial aggregation assay, or in a TLR4 inhibition assay. In some embodiments, the pharmaceutical composition can be an aqueous solution, a suspension, or a solid form. In some embodiments, the pharmaceutical composition of rhSP-D is suitable for lyophilization to a solid form. In some embodiments, a solid form, such as a lyophile or powder, can be administered to a lung, and/or can be reconstituted to form a certain solution suitable for administration to a lung. In some embodiments, the pharmaceutical composition comprising the aqueous solution or suspension of rhSP-D is suitable for administration to a lung.

Certain activities of rhSP-D can be readily determined using bacterial aggregation assays, Toll-like receptor 4 (TLR4) inhibition assays, and/or an asymmetric flow field-flow fractionation with multi-angle laser light scattering (AF4-MALLS) analysis. In some embodiments, the activity of rhSP-D can include a biological activity, such as activity measured in a bacterial aggregation assays, or a TLR4 inhibition assay. In some embodiments, the activity of rhSP-D can include the activity of a population of the rhSP-D to form certain oligomeric forms of the rhSP-D and/or to form a certain distribution of oligomeric forms of the rhSP-D. Example methods to identify the distribution of oligomeric forms of rhSP-D in a sample are provided in WO 2019/191254.

In some embodiments, the pharmaceutical composition can include a buffer. Examples of buffers include acetate, citrate, glutamate, histidine, succinate, and phosphate. In some embodiments, the buffer is histidine. In some embodiments, the concentration of the buffer, such as histidine, is 1 mM, 2 mM, 3 mM, 4mM, 5 mM, 6 mM, 7 mM, 8 mM, 9 mM, 10 mM, or a concentration in a range between any two of the foregoing concentrations. In some embodiments, the concentration of the buffer, such as histidine, is about 1 mM, about 2 mM, about 3 mM, about 4 mM, about 5 mM, about 6 mM, about 7 mM, about 8 mM, about 9 mM, about 10 mM, or a concentration in a range between any two of the foregoing concentrations.

In some embodiments, the pharmaceutical composition can include a sugar. Examples of sugars include trehalose, sucrose, maltose, lactose, glucose, fructose, galactose, mannose, arabinose, xylose, ribose, rhamnose, trehalose, sorbose, melezitose, raffinose, thioglucose, thiomannose, thiofructose, octa-O-acetyl-thiotrehalose, thiosucrose, and thiomaltose. In some embodiments, the sugar is lactose. In some embodiments, the concentration of the sugar, such as lactose, is 200 mM, 250 mM, 265 mM, 300 mM, or a concentration in a range between any two of the foregoing concentrations. In some embodiments, the concentration of the sugar, such as lactose, is about 200 mM, about 250 mM, about 265 mM, about 300 mM, or a concentration in a range between any two of the foregoing concentrations.

In some embodiments, the pharmaceutical composition can include a calcium salt. Examples of calcium salts include calcium chloride, calcium bromide, calcium acetate, calcium sulfate, and calcium citrate. In some embodiments, the calcium salt is calcium chloride. In some embodiments, the concentration of the calcium salt, such as calcium chloride, is 1 mM, 2 mM, 3 mM, 4 mM, 5 mM, 6 mM, 7 mM, 8 mM, 9 mM, 10 mM, or a concentration in a range between any two of the foregoing concentrations. In some embodiments, the concentration of the calcium salt, such as calcium chloride, is about 1 mM, about 2 mM, about 3 mM, about 4 mM, about 5 mM, about 6 mM, about 7 mM, about 8 mM, about 9 mM, about 10 mM.

In some embodiments, the pharmaceutical composition can include an inorganic salt or organic salt. Examples of inorganic salts include sodium chloride, potassium chloride, calcium chloride, sodium phosphate, potassium phosphate, and sodium hydrogen carbonate. Examples of organic salts include sodium citrate, potassium citrate and sodium acetate. In some embodiments, the inorganic salt is sodium chloride. In some embodiments, the concentration of the inorganic salt or organic salt, such as sodium chloride, is 0.1 mM, 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM, 100 mM, or a concentration in a range between any two of the foregoing concentrations. In some embodiments, the concentration of the inorganic salt or organic salt, such as sodium chloride, is about 0.1 mM, about 1 mM, about 2 mM, about 3 mM, 4 mM, about 5 mM, about 6 mM, about 7 mM, about 8 mM, about 9 mM, about 10 mM, about 20 mM, about 30 mM, about 40 mM, about 50 mM, about 60 mM, about 70 mM, about 80 mM, about 90 mM, about 100 mM, or a concentration in a range between any two of the foregoing concentrations. In some embodiments, the pharmaceutical composition can lack an inorganic salt or organic salt, such as sodium chloride.

In some embodiments, the pharmaceutical composition can include a surface-active agent. Examples of surface-active agents include hexadecanol, tyloxapol, dipalmitoylphosphatidylcholine (DPPC), PG, palmitoyl-oleoyl phosphatidylglycerol, palmitic acid, tripalmitin, polysorbates such as polysorbate-20, polysorbate-80, polysorbate-21, polysorbate-40, polysorbate-60, polysorbate-65, polysorbate-81, and polysorbate-85. More examples of surface active agents include poloxamer such as poloxamer 188, Triton such as Triton X-100, sodium dodecyl sulfate (SDS), sodium laurel sulfate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine,cetyl-betaine, lauroamidopropyl-betaine, cocamidopropyl-, linoleamidopropyl-betaine, myristamidopropyl-betaine, palmidopropyl-betaine, isostearamidopropyl-betaine, myristamidopropyl-dimethylamine, palmidopropyl-dimethylamine,isostearamidopropyl-dimethylamine, sodium methyl cocoyl-taurate, disodium methyl oleyl-taurate, polyethyl glycol, polypropyl glycol, and copolymers of ethylene and propylene glycol. In some embodiments, the surface-active agent is tyloxapol. In some embodiments, the concentration of the surface-active agent, such as tyloxapol, is 0.0001%, 0.0005%, 0.001%, 0.005%, 0.01%, 0.05%, 0.1%, 0.5%, 1%, (v/v) or a concentration in a range between any two of the foregoing concentrations. In some embodiments, the concentration of the surface-active agent, such as tyloxapol, is about 0.0001%, about 0.0005%, about 0.001%, about 0.005%, about 0.01%, about 0.05%, about 0.1%, about 0.5%, about 1%, (v/v) or a concentration in a range between any two of the foregoing concentrations. In some embodiments, the pharmaceutical composition can lack a surface-active agent, such as tyloxapol.

In some embodiments, the pharmaceutical composition can have a pH of 5.0, 5.5, 6.0, 6.5, 7.0, or a pH in a range between any two of the foregoing values. In some embodiments, the pharmaceutical composition can have a pH of about 5.0, about 5.5, about 6.0, about 6.5, about 7.0, or a pH in a range between any two of the foregoing values.

In some embodiments, the concentration of rhSP-D in the pharmaceutical composition can be 0.1 mg/ml, 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, or a concentration in a range between any two of the foregoing concentrations. In some embodiments, the concentration of protein, such as rhSP-D , in the pharmaceutical composition can be about 0.1 mg/ml, about 0.5 mg/ml, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, or a concentration in a range between any two of the foregoing concentrations.

In some embodiments, the pharmaceutical composition can include a bulking agent. Examples of bulking agents include a sugar disclosed herein. More examples of bulking agents include mannitol, xylitol, sorbitol, maltitol, lactitol, glycerol, erythritol, arabitol, glycerine, glycine, alanine, threonine, valine, and phenylalanine. In some embodiments, the concentration of the bulking agent, is 0.1 mM, 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM, 100 mM, or a concentration in a range between any two of the foregoing concentrations. In some embodiments, the concentration of the bulking agent, is about 0.1 mM, about 1 mM, about 2 mM, about 3 mM, 4 mM, about 5 mM, about 6 mM, about 7 mM, about 8 mM, about 9 mM, about 10 mM, about 20 mM, about 30 mM, about 40 mM, about 50 mM, about 60 mM, about 70 mM, about 80 mM, about 90 mM, about 100 mM, or a concentration in a range between any two of the foregoing concentrations.

In some embodiments, the pharmaceutical composition can include a chelating agent. In some embodiments, the pharmaceutical composition can lack a chelating agent. Examples of chelating agents include EDTA, and EGTA.

In some embodiments, the rhSP-D comprises a wild-type human SP-D polypeptide. In some embodiments, the rhSP-D includes a polymorphism of the human SP-D polypeptide. Example SP-D polypeptide sequences are provided in TABLE 1. Polymorphisms in the human SP-D polypeptide can include: residue 11, ATG (Met) -> ACG (Thr); residue 25, AGT (Ser) -> AGC (Ser); residue 160, ACA (Thr) -> GCA (Ala); residue 270, TCT (Ser) -> ACT (Thr); and residue 286, GCT (Ala) -> GCC (Ala) in which the positions relate to a position in a mature SP-D polypeptide, such as the example polypeptide of SEQ ID NO:02. In some embodiments, the rhSP-D comprises a certain residue at a polymorphic position in which the residue selected from Met11/31, Thr160/180, Ser 270/290, and Ala 286/306 in which residue positions relate to a position in the mature SP-D polypeptide, such as example SEQ ID NO:02, and a position in the SP-D polypeptide with its leader polypeptide, such as example SEQ ID NO:01. In some embodiments, the rhSP-D comprises Met11/31. In some embodiments, the rhSP-D comprises Met11/31, Thr160/180, Ser 270/290, and Ala 286/306. In some embodiments, the rhSP-D polypeptide has an identity with a polypeptide of SEQ ID NO:02 over the entire length of the polynucleotide of at least 80%, 90%, 95%, 99% and 100%, or any percentage in a range between any of the foregoing percentages.

**TABLE 1**

| **SEQ ID NO.** | **Sequence** |
|---|---|
| SEQ ID NO:01 | MLLFLLSALVLLTQPLLGYLEAEMKTYSHRTMPSACTLV MCSSVESGLPGRDGRDGREGPRGEKGDPGLPGAAGQAG MPGQAGPVGPKGDNGSVGEPGPKGDTGPSGPPGPPGVPG PAGREGPLGKQGNIGPQGKPGPKGEAGPKGEVGAPGMQG SAGARGLAGPKGERGVPGERGVPGNTGAAGSAGAMGPQ GSPGARGPPGLKGDKGIPGDKGAKGESGLPDVASLRQQV EALQGQVQHLQAAFSQYKKVELFPNGQSVGEKIFKTAGF VKPFTEAQLLCTQAGGQLASPRSAAENAALQQLVVAKNE AAFLSMTDSKTEGKFTYPTGESLVYSNWAPGEPNDDGGS EDCVEIFTNGKWNDRACGEKRLVVCEF |
| SP-D polypeptide including a leader sequence (underlined) and polymorphisms (underlined) at: Met 31, Thr 180, Ser 290, Ala 306. | |
| SEQ ID NO:02 | AEMKTYSHRTMPSACTLVMCSSVESGLPGRDGRDGREGP RGEKGDPGLPGAAGQAGMPGQAGPVGPKGDNGSVGEPG PKGDTGPSGPPGPPGVPGPAGREGPLGKQGNIGPQGKPGP KGEAGPKGEVGAPGMQGSAGARGLAGPKGERGVPGERG VPGNTGAAGSAGAMGPQGSPGARGPPGLKGDKGIPGDKG AKGESGLPDVASLRQQVEALQGQVQHLQAAFSQYKKVEL FPNGQSVGEKIFKTAGFVKPFTEAQLLCTQAGGQLASPRS AAENAALQQLVVAKNEAAFLSMTDSKTEGKFTYPTGESL VYSNWAPGEPNDDGGSEDCVEIFTNGKWNDRACGEKRL VVCEF |
| SP-D polypeptide of SEQ ID NO:01, without leader sequence, and polymorphisms (underlined) at: Met 11, Thr 160, Ser 270, Ala 286. | |

In some embodiments, the rhSP-D is derived from a human myeloid leukemia cell line expressing the rhSP-D from an integrated transgene. Example expression vectors, rhSP-D polypeptides, cell-lines, and methods of purifying rhSP-D from such cells, are provided in U.S Patent Publications 2019/0071693 and U.S. 2019/0071694.

In some embodiments, a pharmaceutical composition, such as a solution or suspension, comprising a population of rhSP-D polypeptides can have a certain distribution of oligomeric forms of the rhSP-D. A composition of rhSP-D can include different rhSP-D oligomeric forms including: trimers with a mass of about 130-150 kDa on SDS-PAGE which include 3 monomers and which together can have a rod-like appearance as visualized by atomic force microscopy (AFM); hexamers with a mass of about 250 kDa on SDS-PAGE which include 6 monomers; dodecamers with a predicted mass of about 520 kDa, as measured by AF4-MALLS and which include 12 monomers and can have an X-like appearance as visualized by AFM; larger heterogeneous oligomeric species which comprise multiples of more than four trimers and can have a star-like- or star- shaped appearance with a radius of about 70 nm as visualized and identified by AFM, such oligomers are known as star-like oligomers; and even larger oligomeric species having a radius larger than 70 nm as visualized by AFM and measured by AF4-MALLS and known as aggregates.

In some embodiments, more than about 10%, 20%, 30%, 50%, 60%, 70%, 80%, 90%, or a percentage within a range between any two of the foregoing percentages, of the oligomeric forms of rhSP-D can be a dodecameric oligomeric form of rhSP-D as measured as a relative peak area (RPA) in an AF4-MALLS analysis. In some embodiments, more than about 10%, 20%, 30%, 50%, 60%, 70%, 80%, 90%, or a percentage within a range between any two of the foregoing percentages, of the mass of the oligomeric forms, such as in a solution or suspension, of rhSP-D can be a dodecameric oligomeric form of rhSP-D. In some embodiments, more than about 10%, 20%, 30%, 50%, 60%, 70%, 80%, 90%, or a percentage within a range between any two of the foregoing percentages, of the number of molecules of the oligomeric forms, such as in a solution or suspension, of rhSP-D can be a dodecameric oligomeric form of rhSP-D.

In some embodiments, less than about 0.5%, 1%, 2%, 3%, 4%, 5%, 10%, 20%, 30%, 50%, or a percentage within a range between any two of the foregoing percentages, of the oligomeric forms of rhSP-D can be an aggregate oligomeric form of rhSP-D as measured as an RPA or an adjusted RPA in an AF4-MALLS analysis. In some embodiments, less than about 0.5%, 1%, 2%, 3%, 4%, 5%, 10%, 20%, 30%, 50%, or a percentage within a range between any two of the foregoing percentages, of the mass of the oligomeric forms, such as in a solution or suspension, of rhSP-D can be an aggregate oligomeric form of rhSP-D. In some embodiments, less than about 0.5%, 1%, 2%, 3%, 4%, 5%, 10%, 20%, 30%, 50%, or a percentage within a range between any two of the foregoing percentages, of the number of molecules of the oligomeric forms, such as in a solution or suspension, of rhSP-D can be an aggregate oligomeric form of rhSP-D.

In some embodiments, a pharmaceutical composition consists of, consists essentially of, or comprises 1 mg/ml rhSP-D, 5 mM histidine, 265 mM lactose, 5 mM calcium chloride, having a pH of 6.0. In some embodiments, a pharmaceutical composition consists of, consists essentially of, or comprises 1 mg/ml rhSP-D, 5 mM histidine, 265 mM lactose, 1 mM calcium chloride, having a pH of 6.0. In some embodiments, a pharmaceutical composition consists of, consists essentially of, or comprises 2 mg/ml rhSP-D, 5 mM Histidine, 265 mM Lactose, 1 mM CaCl₂, pH 6.0. In some embodiments, a pharmaceutical composition consists of, consists essentially of, or comprises 2 mg/ml rhSP-D, 5 mM histidine, 265 mM lactose, 5 mM calcium chloride, having a pH of 6.0. In some embodiments, a pharmaceutical composition consists of, consists essentially of, or comprises 4 mg/ml rhSP-D, 5 mM histidine, 265 mM lactose, 5 mM calcium chloride, having a pH of 6.0.

In some embodiments, the pharmaceutical compositions provided herein can include an admixture with a suitable carrier, diluent, or excipient such as sterile water, physiological saline, glucose, or the like, and can contain auxiliary substances such as wetting or emulsifying agents, pH buffering agents, gelling or viscosity enhancing additives, preservatives, flavoring agents, colors, and the like, depending upon the route of administration and the preparation desired. *See e.g.,* "Remington: The Science and Practice of Pharmacy", Lippincott Williams & Wilkins; 20th edition (June 1, 2003) and "Remington's Pharmaceutical Sciences," Mack Pub. Co.; 18th and 19th editions (December 1985, and June 1990, respectively). In some embodiments, such preparations can include complexing agents, metal ions, polymeric compounds such as polyacetic acid, polyglycolic acid, hydrogels, dextran, and the like, liposomes, microemulsions, micelles, unilamellar or multilamellar vesicles, erythrocyte ghosts or spheroblasts. Suitable lipids for liposomal formulation include monoglycerides, diglycerides, sulfatides, lysolecithin, phospholipids, saponin, bile acids, and the like. The presence of such additional components can influence the physical state, solubility, stability, rate of *in vivo* release, and rate of *in vivo* clearance, and are thus can be chosen according to the intended application, such that the characteristics of the carrier are tailored to the selected route of administration, such as pulmonary delivery, such as delivery to a lung, such as delivery to a neonate lung.

In some embodiments, pharmaceutical compositions are suitable for intratracheal, intrabronchial or bronchoalveolar administration to a lung. In some embodiments, intratracheal, intrabronchial or bronchoalveolar administration can include spraying, lavage, inhalation, flushing or installation, using as fluid a physiologically acceptable composition in which the pharmaceutical composition has been dissolved. Methods of administration can include the use of continuous positive airway pressure (CPAP). Methods of administration can include direct intubation. In some embodiments, pharmaceutical compositions provided herein can be delivered to the lungs while inhaling. Example forms that can be delivered include dry powders, and aerosols. A wide range of mechanical devices designed for pulmonary delivery of therapeutic products can be employed, including but not limited to nebulizers, metered dose inhalers, and powder inhalers, all of which are familiar to those skilled in the art. These devices employ formulations suitable for the dispensing of a pharmaceutical composition. Typically, each formulation is specific to the type of device employed and can involve the use of an appropriate propellant material, in addition to diluents, adjuvants, and/or carriers useful in therapy.

### Kits

In a non-claimed embodiment is provided a kit. In some embodiments, a kit can include a pharmaceutical composition provided herein. Some embodiments include a sterile container comprising a pharmaceutical composition provided herein. Some embodiments include a pharmaceutical composition provided herein in lyophilized form, and a sterile reconstituting solution. In some embodiments, a kit can include a device for administering a pharmaceutical composition provided herein, such as an inhaler, and a nebulizer.

### EXAMPLES

### Example 1-In vitro binding of S-protein to immobilized rhSP-D

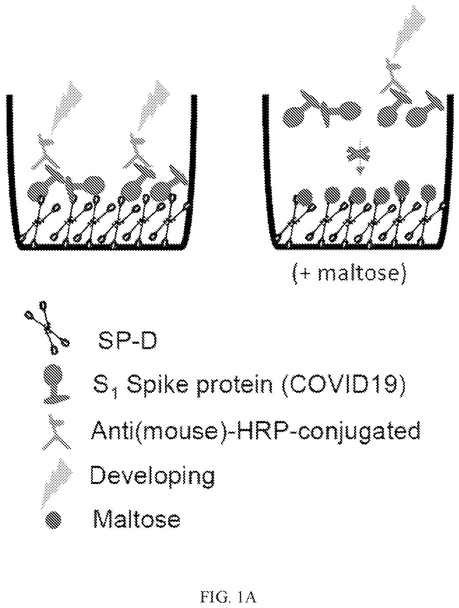

An ELISA based binding assay was developed to determine the binding of immobilized recombinant human SP-D (rhSP-D) to an S1 subunit of a spike protein of SARS-CoV-2 (S1-protein). SP-D binding activity is enhanced by the presence of calcium, and SP-D binds maltose. Assays were performed in the presence of calcium; in the presence of a calcium chelator, EDTA; or in the presence of maltose. FIG. 1A depicts a schematic overview of the assay.

Recombinant S1-protein was produced in HEK293 cells with a mouse Fc IgG tag on the C-terminal end (SinoBiologicals, #40591-V05H1). A first sample of rhSP-D was produced from human myeloid leukemia cells; and a second sample of rhSP-D was obtained from CHO cells. The wells of microtiter plates were coated with 200 µL of a suspension of rhSP-D at 5 µg/mL or at 2 µg/mL in a carbonate-bicarbonate coating buffer (50 mM NaHCO₃-Na₂CO₃ (pH 9.6)). The plate was incubated overnight at 4 °C. Plates were washed 5 times between incubations and all washes and dilutions from this point were carried out with dilution buffer: 0.05% TBS-tween, 5 mM CaCl₂ (TBS is 50 mM Tris pH 7.4, 150 mM NaCl). Washes were performed by adding 200 µL/well of washing buffer followed by aspiration of the wells, this process was repeated 5 times. After washing the plate, wells were blocked with 2% bovine serum albumin (BSA) in dilution buffer (200 µL/well) for 1 hour at room temperature to avoid unspecific binding of rhSP-D to uncoated areas of the well. The plate was washed and samples of serial diluted (1:2) S-protein SARS-CoV-2 (from 10 µg/mL to 9.8 ng/mL) were added to the wells to obtain a standard curve.

To determine if the binding was mediated by the carbohydrate recognition domain of rhSP-D, a second set of S1-protein samples was prepared where maltose was added to the S1-protein samples to obtain a final concentration of 200 mM maltose and it was incubated 10 minutes before being added to the plate wells. A third set of S1-protein samples was prepared with the same purpose, in this case, using 100 mM EDTA in the dilution buffer instead of 5 mM calcium to inhibit the calcium-dependent binding of rhSP-D. In all the cases, once added to the wells, the S1-protein was incubated for 1 hour at room temperature.

After washing the plate, 100 µL of anti-mouse IgG horseradish peroxidase (HRP)-conjugated antibody (dilution 1:5000) (#7076, Cell Signaling; Danvers, MA, USA) were incorporated and incubated for 1 hour at room temperature. Plates were washed and 100 µL of TMB/E (3,3',5,5'-tetramethybenzidine) (#TMBS010001, Surmodics) were added and incubated at room temperature for 10 minutes and the reaction was stopped with 100 µL of 2N H₂SO₄. Plates were read for absorption at 450 nm.

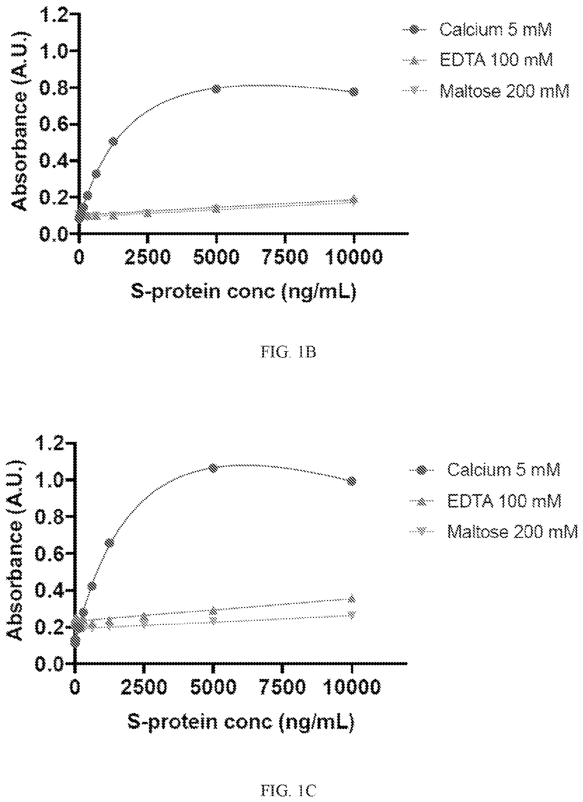

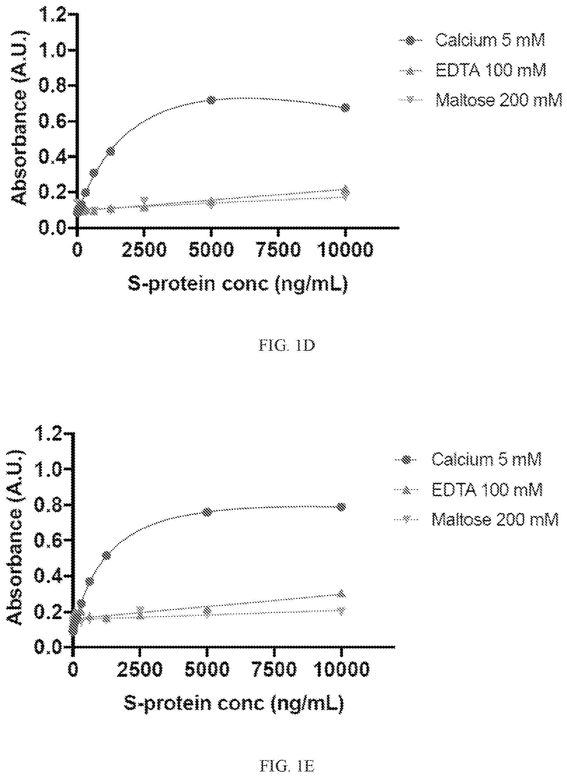

FIG. 1B and FIG. 1C summarize results for wells coated with solutions of rhSP-D at 5 µg/mL, for a first sample of SP-D and a second sample of SP-D, respectively. FIG. 1D and FIG. 1E summarize results for wells coated with solutions of rhSP-D at 2 µg/mL, for a first sample of SP-D and a second sample of SP-D, respectively. The S1-protein bound to SP-D in the presence of calcium. The binding was inhibited by the presence of EDTA or maltose. Thus, the S1-protein bound to SP-D in a calcium dependent manner, and this binding was inhibited by a competitor, maltose.

### Example 2-In vitro binding of rhSP-D to immobilized S-protein

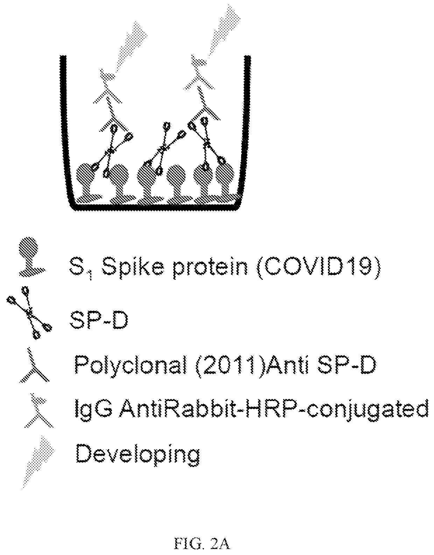

An ELISA based binding assay was developed to determine the binding of rhSP-D to an immobilized S1 subunit of a spike protein of SARS-CoV-2 (S1-protein). Assays were performed in the presence of calcium; or in the presence of a calcium chelator, EDTA. FIG. 2A depicts a schematic overview of the assay.

Recombinant S1-protein was produced in HEK293 cells with a mouse Fc IgG tag on the C-terminal end (SinoBiologicals, #40591-V05H1). A first sample of rhSP-D was produced from human myeloid leukemia cells; and a second sample of rhSP-D was obtained from CHO cells. The wells of microtiter plates were coated with 200 µL of a suspension of S1-protein at 2.5 µg/mL in a carbonate-bicarbonate coating buffer (50 mM NaHCO₃-Na₂CO₃ (pH 9.6)). The plate was incubated overnight at 4 °C. Plates were washed 5 times between incubations and all washes and dilutions from this point were carried out with dilution buffer: 0.05% TBS-tween, 5 mM CaCl₂. Washes were performed by adding 200 µL/well of washing buffer followed by aspiration of the wells, this process was repeated 5 times. After washing the plate, wells were blocked with 2% BSA in dilution buffer (200 µL/well) for 1 hour at room temperature to avoid unspecific binding of rhSP-D to uncoated areas of the well. The plate was washed as described and samples of serial diluted (1:2) rhSP-D (from 5 µg/mL to 4.9 ng/mL) were added to the wells to obtain a standard curve.

To determine if the binding was mediated by the carbohydrate recognition domain of rhSP-D, a second set of rhSP-D samples was prepared using 100 mM EDTA in the dilution buffer instead of 5 mM calcium to inhibit the calcium-dependent binding of rhSP-D. In all the cases, once added to the wells, the rhSP-D was incubated for 1 hour at room temperature. After washing the plate, 50 µL of rabbit anti-SP-D antibody (dilution 1:5000) were incorporated and incubated for 1 hour at room temperature.

The plate was washed and 100 µL of anti-rabbit IgG horseradish peroxidase (HRP)-conjugated antibody (dilution 1:7500) (#7074, Cell Signaling; Danvers, MA, USA) were incorporated and incubated for 1 hour at room temperature. Plates were washed and 100 µL of TMB/E (3,3',5,5'-tetramethybenzidine) (#TMBS010001, Surmodics) were added and incubated at room temperature for 5 minutes and the reaction was stopped with 100 µL of 2N H2SO4. Plates were read for absorption at 450 nm.

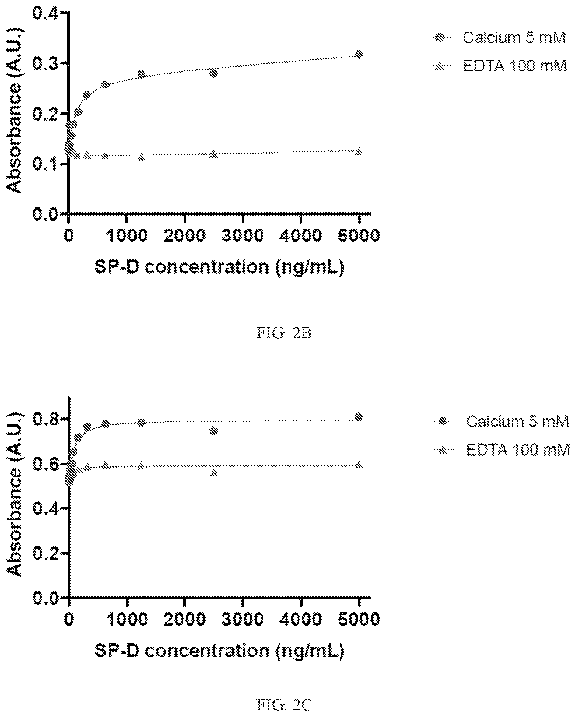

FIG. 2B and FIG. 2C summarize results for wells coated with S1-protein, for a first sample of SP-D and a second sample of SP-D, respectively. The S1-protein bound to SP-D in the presence of calcium. The binding was inhibited by the presence of EDTA. Thus, the S1-protein bound to SP-D in a calcium dependent manner.

### Example 3-Pulmonary SP-D concentration in COVID-19 patients

This example shows determination of SP-D levels in bronchoalveolar lavage of COVID-19 patients. Bronchoscopy and bronchoalveolar lavage fluid (BALF) was obtained as described in Pandolfi, L., et al., (2020) BMC Pulm Med 20: 301. Briefly, bronchoscopies were performed in sedated, paralyzed and mechanically ventilated patients (n = 12) with COVID-19 confirmed by a PCR test. BALF aliquots were collected after 5-6 bolus of 20 mL sterile saline, the initial 20 mL were discarded. The suspensions were centrifuged at 400 g for 10 min and supernatants were inactivated with 0.2% SDS, 0.1% Tween20 followed by 15 min at 65 °C. The resulting BALF were stored at -20 °C until analysis. SP-D levels in BALF were quantified by an ELISA procedure using human anti-SP-D antibodies (Biovendor). BALF was collected after authorization by Ethic Committee of Ospedale Luigi Sacco (experimentation number 2020/ST/145). Bronchoalveolar lavage samples were collected from COVID-19 patients with different age, characteristics and comorbidities, and are indicated in TABLE 2. Body mass index (BMI) above 30 considered obesity. The comorbidities that were screened included: smoking, cardiovascular disease (CV), respiratory disease, immunosuppression, human immunodeficiency virus (HIV), diabetes mellitus type I and type II, and cancer.

**TABLE 2**

| **Age** | **Sex** | **BMI** | **Collection of BALF sample (time)** | | **Comorbidity** |
|---|---|---|---|---|---|
| | | | Hospitalization day | Intubation day | |
| 28 | F | 26.6 | 9 | 3 | N |
| 40 | M | 44.2 | 11 | 10 | N |
| 46 | F | 29.4 | 14 | 14 | N |
| 50 | M | 34.6 | 3 | 3 | Y (HIV) |
| 53 | M | 26.2 | 11 | 9 | Y (smoker) |
| 55 | M | 24.8 | 24 | 13 | Y (smoker, CV, cancer) |
| 60 | M | - | 4 | 3 | Y (CV) |
| 61 | M | 21.6 | 6 | 5 | Y (cancer) |
| 64 | M | 32.8 | 6 | 6 | Y (CV) |
| 68 | F | 23.4 | 9 | 9 | N |
| 68 | M | 25.2 | 50 | 50 | N |
| 73 | M | 29.4 | 4 | 3 | N |
| 28 | F | 26.6 | 9 | 3 | N |
| 40 | M | 44.2 | 11 | 10 | N |
| Sex: M (male), F (female) | | | | | |
| BMI (body mass index) | | | | | |
| Absence of a comorbidity is indicated as "N", presence of them is indicated with "Y" with indicating the comorbidity in brackets | | | | | |

Decreased SP-D levels have been found in the bronchoalveolar lavage of several respiratory diseases that exhibit acute lung injury (Sorensen, G. L., et al., (2007) Immunobiology 212:381-416). The pulmonary levels of SP-D in COVID-19 patients was found to have a median concentration of 68.9 ng/mL (mean=244.8 ng/mL, n=12) (FIG. 3). This compares to BALF SP-D levels in non-COVID-19 healthy control subjects that has previously been reported to be 900-1300 ng/mL and in surviving (940 ng/ml) and non-surviving (406 ng/ml) early ARDS patients (Hermans C, et al (1999). Am J Respir Crit Care Med 159:646-678; and Honda Y, et al. (1995) Am J Respir Crit Care Med 152:1860-1866). Therefore, COVID-19 patients were found to have decreased pulmonary SP-D levels when compared to levels reported in the literature for healthy subjects and ARDS patients.

### Example 4-Recombinant hSP-D binds to the S-protein of SARS-CoV-2

Binding experiments substantially similar to those described in Examples 1-2 listed above were performed. Full length recombinant human rhSP-D was produced in a human cell line GlycoExpress^{®} (GEX) developed in Glycotope-GmbH. The rhSP-D variant was Met¹¹, Thr¹⁶⁰, Ser²⁶⁰. The purification process of rhSP-D has been described elsewhere (Ikegami, M,., et al., ( 2006) Am J Respir Crit Care Med 173:1342-1347; and Arroyo, R., et al., (2018) J Mol Biol 430:1495-1509). Recombinant SARS-CoV-2 spike protein variants (S1-subunit) and recombinant human ACE2 protein were expressed in HEK293 cells and purchased from SinoBiologicals (#40591-V08H, #40591-V05H1, #10108-H05H, #40591-V08H3, #40591-V08H10), Acro Biosystems (#S1N-C52H3, #S1N-C52Hk, #S1NN-C52Hg), The NativeAntigen Company (#REC31806-100-HRP) and from Biomart Creative (#ACE2-736H).

Briefly, a first ELISA assay was developed in which microtiter plates were coated with a S1-spike-protein variant (0.4 µg in 200 µL/well). Washes and dilutions were performed with 0.05% TBS-tween, 5 mM CaCl₂. Wells were blocked with 2% BSA and serially diluted rhSP-D (10 µg/mL to 9.8 ng/mL) was added to the wells. Bound rhSP-D was detected with a mouse anti-SP-D antibody (#2D12-A-88, Seven Hills Bioreagents), followed by an anti-mouse IgG horseradish peroxidase (HRP)-conjugated antibody (#7076, Cell Signaling). The plates were developed with TMB (#TMBS010001, Surmodics) for 10 minutes and the reaction was stopped with 2N H₂SO₄. Plates were read for absorption at 450 nm. Non-binding negative controls were included, using 50 mM EDTA to prevent calcium-dependent binding or 200 mM maltose also with 5 mM calcium to create binding competition between maltose and S1-protein. To address nonspecific binding to the plate, wells were coated with 1% BSA instead of S1-protein.

A second ELISA assay was also developed in which the wells were coated with rhSP-D instead of S1-protein. Serially diluted S1-protein samples with a mouse Fc tag (10 µg/mL to 9.8 ng/mL) were added to the wells. Bound S1-protein was detected with the same anti-mouse IgG HRP-conjugated antibody. Analysis of the binding isotherms was performed with GraphPad Prism 8, considering total binding and one site to determine the apparent dissociation constant (kd) and the apparent maximum number of binding sites (Bₘₐₓ).

The ELISA assay indicated that rhSP-D recognized and bound to the subunit S1 of the spike protein from the first identified variant of SARS-CoV-2 (Wuhan variant) with a similar apparent dissociation constant when rhSP-D was the ligand (Kd=1.65) (FIG. 4A) or S1-protein was the ligand (Kd=2.02) (FIG. 4B). The apparent number of maximum binding sites was higher when rhSP-D was the ligand (Bₘₐₓ=1.35, FIG. 4A) compared to S1-protein (Bₘₐₓ=0.81, FIG. 4B), which was expected because the higher order oligomeric forms of rhSP-D (dodecamers and multimers) have several trimeric carbohydrate recognition domains (CRD), the binding site of rhSP-D, while the S1-protein has only one. Binding of rhSP-D to S1-protein was inhibited by EDTA confirming that it was calcium-dependent. Binding competition with maltose, which also binds to the CRD of rhSP-D in a calcium-dependent manner, abrogated the binding of rhSP-D to S-protein. The binding of rhSP-D to S1-protein in the presence of calcium was significantly different (p<0.0001) to the binding with EDTA or maltose strongly suggesting that the CRD of rhSP-D mediates the binding to the carbohydrates described on the S1-protein of SARS-CoV-2.

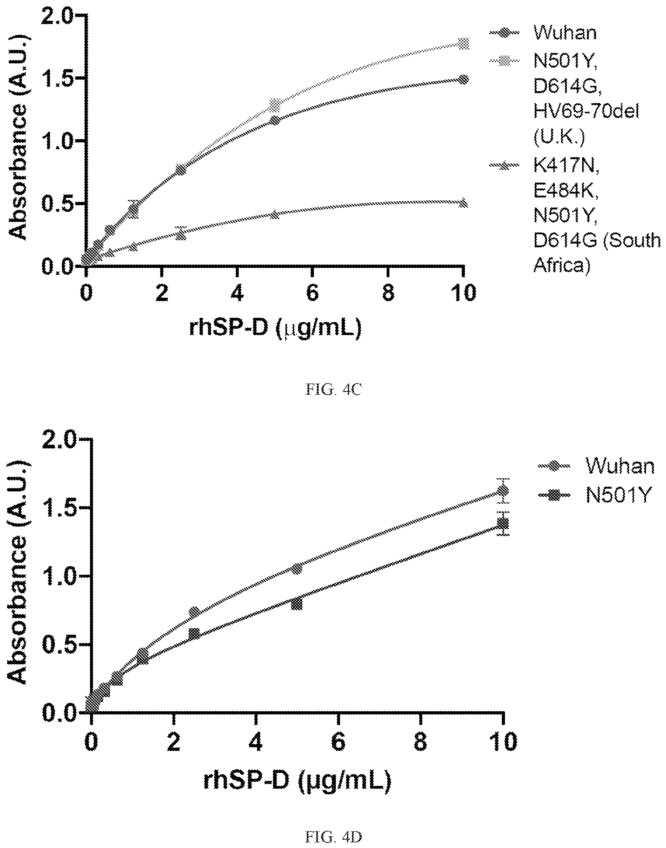

Binding of rhSP-D to the S1-protein bearing the mutations identified in the U.K. B.1.1.7. variant (HV69-70, N501Y, D614G) or in the South African B.1.351 variant (K417N, E484K, N504Y, D614G) was tested. rhSP-D bound to all the variants tested (FIG. 4C). rhSP-D binding to the S1-protein from the U.K. variant was similar to the Wuhan variant, however, binding was significantly decreased with the South African S1-protein variant. Specifically, binding to the South African variant was significantly decreased compared to the Wuhan (p=0.0002) and the U.K. variant (p=0.007), no significant differences observed when comparing Wuhan and U.K. variant (p>0.99) (Friedman Test with Dunn's post hoc).

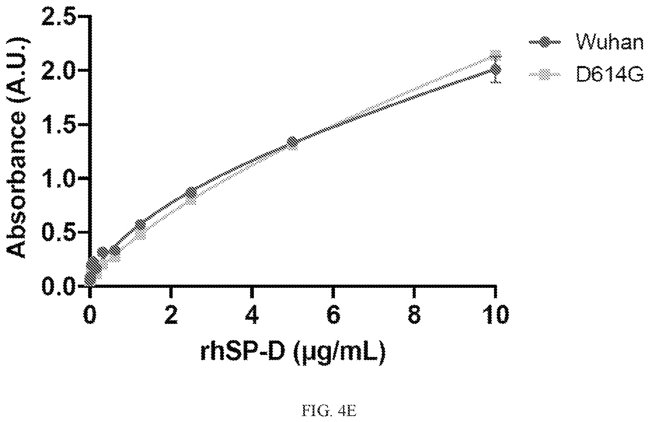

The significance for rhSP-D binding of the two common mutations of the S1-protein, N501Y and D614G, found in the new variants was addressed individually. The mutation N501Y decreased rhSP-D binding when compared to the Wuhan original variant (FIG. 4D), on the other hand the D614G had almost no effect in rhSP-D binding to the spike protein when compared to the Wuhan variant (FIG. 4E). Binding of rhSP-D to the S1-protein variant from Wuhan compared to a S1-protein with a single mutation N501Y (p=0.04) or to D614G (D) (p=0.05) (t-test).

The following experiments were performed with the S1-protein from the Wuhan variant.

### Example 5-rhSP-D forms protein bridges with the S-protein of SARS-CoV-2

To determine if rhSP-D could aggregate SARS-CoV-2, the ability of rhSP-D to link S-protein to a second molecule (maltose-coated beads) was examined. A protein-bridge (aggregation) assay was performed and included a pre-mix approach (FIG. 5A), and a 1^{st} rhSP-D approach (FIG. 5B).

In the pre-mix approach (FIG. 5A), rhSP-D (2 µg or 4 µg) and S1-protein (2 µg; Wuhan variant) were pre-mixed and incubated for 2 hours to favor binding and aggregation of S1-protein by rhSP-D. Then, the mix was added to the beads. After incubation at room temperature for 30 min, the beads were centrifuged and the supernatant (S1) was saved. Then, the beads were washed and eluted as previously described, saving the eluted fraction (P) for analysis.

In the first rhSP-D approach (FIG. 5B), rhSP-D (2 µg or 4 µg) was incubated at room temperature for 30 min with maltose-coated agarose beads in 50 µL TBS (150 mM NaCl, 20 mM Tris (pH 7.4))-10 mM CaCl₂ buffer. The supernatant (S1) with the excess unbound rhSP-D was separated by centrifugation and saved. The beads were washed with TBS-CaCl₂. Then, 2 µg of S1-protein or buffer (negative control) was added to the beads and the final volume was adjusted to 50 µL with TBS-CaCl₂, or with 20 mM TBS-EDTA in the non-binding control. After incubation at room temperature for 2 hours, the beads were centrifuged and the supernatant (S2) was saved. The beads (pellet) were washed with the appropriate buffer followed by elution of the bound rhSP-D with TBS-EDTA 20 mM. The eluted fraction from the pellet (P) was saved for analysis.

In both methods, the presence of rhSP-D and S1-protein in fractions (S1, S2 and P) was determined by SDS-PAGE under reducing conditions and developed by silver staining. Intensity of rhSP-D bands from the samples that contained 4 µg of rhSP-D was quantified by densitometry in duplicate with ImageJ software. The relative intensity of the rhSP-D band in the pellet fraction (P) was calculated considering 100% the intensity of the "S1" band in the buffer control at 5 mM calcium. Densitometry of the gels was performed twice.

The results demonstrated that rhSP-D formed a protein bridge with the Wuhan variant S1-protein ("P" in FIG. 5C: lanes 4 and 8; FIG. 5D: lane 9) and maltose-coated beads. The formation of protein bridges by rhSP-D was was inhibited in the presence of EDTA and therefore calcium-dependent (FIG. 5C: lane 10; FIG. 5D: lane 12). Binding between S-protein and rhSP-D was also confirmed in this second assay because fraction "S2" only contained rhSP-D in the presence of S1-protein (FIG. 5D, lane 2 VS lane 8). The addition of S1-protein to rhSP-D that was previously bound to maltose-coated beads showed that part of that rhSP-D shifted and preferentially bound to the S-protein (observed in "S2" fractions). To determine if rhSP-D could form an aggregate of multiple S-protein and rhSP-D molecules, the pre-mix and 1st-rhSP-D approaches were compared. The pre-mix approach (FIG. 5A) should allow the formation of larger order S-protein and rhSP-D aggregates of multiple S-protein and rhSP-D molecules. In contrast, binding of rhSP-D to maltose beads first, followed by removal of unbound rhSP-D and then binding to S-protein, should be limited to single units of rhSP-D bound to S-protein and maltose (FIG. 5B). The intensity of rhSP-D bands in the eluted ("P")-fraction in the pre-mix approach was stronger than their respective ones in the 1st-SP-D approach (FIG. 5E), which was consistent with the formation of larger order aggregates. Collectively, these data demonstrated the existence of protein bridges facilitated by rhSP-D and suggested the aggregation of SARS-CoV-2 driven by rhSP-D.

### Example 6-S-protein and rhSP-D binding in the presence of ACE2 receptor

The spike protein of SARS-CoV-2 interacts with ACE2 receptors in epithelial cells. Binding of ACE2 to S1-protein (Wuhan variant) in the presence of rhSP-D was examined. Plates were coated with purified S1-protein (Wuhan variant). RhSP-D (0.1 to 1 µg/mL) in TBS-Ca 5 mM or buffer (negative control) were added to the wells and incubated for 2 hours. Without washing, human ACE2 protein (0.186 to 1.5 µg/mL) was added to the wells at each of the rhSP-D concentrations, a control with TBS buffer instead of ACE2 was also included. After incubation for 30 minutes, bound ACE2-mFc was detected with an anti-mouse IgG HRP-conjugated antibody (FIG. 6A, FIG. B). Binding of S1-protein to rhSP-D in the presence of ACE2 was examined. Plates were coated with rhSP-D (5 µL/mL, 200 µL/well). S1-protein HRP-tagged at different concentrations or buffer (negative control), were added to the wells and incubated for 2 hours. Without washing, human ACE2 protein His-tagged was added to the wells to reach 3, 0.375 or 0.045 µg/mL at each of the S1-protein concentrations. After incubation for 30 minutes, bound S1-protein-HRP was detected directly with TMB and the reaction was stopped with 2N H₂SO₄ (FIG. 6C, FIG. D).

A decrease in the binding of ACE2 to S1-protein in the presence of 0.5 µg/mL rhSP-D compared to the control without rhSP-D (FIG. 6A, FIG. 6B) was observed. The results also demonstrated that the addition of ACE2 did not inhibit the binding of rhSP-D to S1-protein (FIG. 6C, FIG. 6D) until a small decrease in binding was observed at the maximum concentration of ACE2 (3 µg/mL). Therefore, rhSP-D and ACE2 bound to different regions of S1-protein allowing the co-interaction of the three molecules.

### Example 7-rhSP-D inhibits SARS-CoV-2 replication in host cells

The effect of rhSP-D on SARS-CoV-2 replication in host cells was tested *in vitro* with a viral replication assay in human Caco-2 cells.

Monolayers of human epithelial Caco-2 cells were prepared 24 hours prior to virus infection in 96-well microtiter plates at 37 °C with 5% CO₂. Growth media was removed from the cells and the rhSP-D was applied and tested in triplicates at eight serial half-log10 dilution concentrations starting at 100 µg/mL. SARS-CoV-2 (strain USA/WA1/2020) at 200 CCID50 (50% cell culture infectious dose) was added to wells designated for virus infection. MOI=0.02. Controls were performed with infected and not treated (virus controls) cells and untreated and uninfected (cell controls) cells. Plates were incubated at 37° C for 72 hours. A sample of supernatant was taken from each infected well for testing and virus titer determination (n=3 replicates). Titration of the viral samples previously collected was performed by endpoint dilution as described in Reed LJ, et al (1938) American Journal of Epidemiology 27:493-497. Serial 10-fold dilutions of virus were made and plated into wells containing fresh cell monolayers of Vero 76 cells. Plates were incubated, and cells were scored for presence or absence of virus after distinct cytopathogenic effect is observed, and the CCID50 calculated using the Reed *et al* method. The 90% (one log10) effective concentration (EC90) was calculated. Cell toxicity of rhSP-D was evaluated in additional plate wells by using a neutral red dye that penetrated into living cells and allows quantification of viable cells. In the cell toxicity assay, the more intense the red color, the larger the number of viable cells present in the wells. The dye content in each well was quantified using a spectrophotometer at 540 nm wavelength.

rhSP-D inhibited viral replication in a dose-dependent manner with higher concentrations of rhSP-D leading to greater inhibition of viral replication, which was observed by measuring the virus titer in the cell supernatant at the different rhSP-D concentrations tested and reported as CCID50 (50% cell culture infectious dose) (FIG. 7). The concentration of rhSP-D necessary to inhibit viral replication by 90% (EC90) was 3.7 µg/mL. Moreover, rhSP-D did not show any cell toxicity even at the highest rhSP-D tested (100 µg/mL) when compared to control (non-treated and non-infected) cells.

### Example 8-Treatment of a SARS-CoV-2 infection with rhSP-D

A patient having a SARS-CoV-2 infection is administered a pharmaceutical solution comprising rhSP-D, 5 mM Histidine, 265 mM Lactose, and 5 mM CaCl₂. The patient has symptoms including fever, cough, shortness of breath, fatigue, muscle pain, diarrhea, sore throat, loss of smell, and abdominal pain. On administration of the pharmaceutical solution, one or more symptoms of the SARS-CoV-2 infection in the patient are reduced.

The term "comprising" as used herein is synonymous with "including," "containing," or "characterized by," and is inclusive or open-ended and does not exclude additional, unrecited elements or method steps.

## Claims

1. A pharmaceutical composition for use in treating or ameliorating a viral infection comprising a coronavirus in a subject, wherein the pharmaceutical composition comprises a recombinant human surfactant protein D (rhSP-D),
wherein the viral infection comprises SARS-CoV-2, and
wherein the SARS-CoV-2 comprises an S1 protein comprising an N501Y mutation or a D614G mutation.

2. The composition for use according to claim 1, wherein the S1 protein lacks a mutation selected from K417N and E484K.

3. The composition for use according to claim 1 or 2, wherein the pharmaceutical composition further comprises a buffer, a sugar, and a calcium salt.

4. The composition for use according to claim 3, wherein the buffer is selected from the group consisting of acetate, citrate, glutamate, histidine, succinate, and phosphate.

5. The composition for use according to claim 3 or 4, wherein the buffer comprises histidine, and wherein the concentration of the histidine is from 1 mM to 10 mM.

6. The composition for use according to any one of claims 3-5, wherein the sugar is selected from the group consisting of sucrose, maltose, lactose, glucose, fructose, galactose, mannose, arabinose, xylose, ribose, rhamnose, trehalose, sorbose, melezitose, raffinose, thioglucose, thiomannose, thiofructose, octa-O-acetyl-thiotrehalose, thiosucrose, and thiomaltose.

7. The composition for use according to any one of claims 3-6, wherein the sugar comprises lactose, and wherein the concentration of the lactose is from 200 mM to 300 mM.

8. The composition for use according to any one of claims 3-7, wherein the calcium salt is selected from the group consisting calcium chloride, calcium bromide, calcium acetate, calcium sulfate, and calcium citrate.

9. The composition for use according to any one of claims 3-8, wherein the calcium salt comprises calcium chloride, and wherein the concentration of the calcium chloride is from 1 mM to 10 mM.

10. The composition for use according to any one of claims 3-9, wherein the pharmaceutical composition has a pH from 5.0 to 7.0.

11. The composition for use according to any one of claims 3-10, wherein the concentration of the rhSP-D is from 0.1 mg/ml to 10 mg/ml.

12. The composition for use according to any one of claims 1-11, wherein the rhSP-D comprises an amino acid sequence having at least 95% identity to an amino acid sequence of SEQ ID NO:02.

13. The composition for use according to any one of claims 1-12, wherein the subject is mammalian.

14. The composition for use according to any one of claims 1-13, wherein the S1 protein comprises an N501Y mutation, a D614G mutation, and an HV69-70 del mutation.

## Patentansprüche

1. Pharmazeutische Zusammensetzung zur Verwendung bei der Behandlung oder Linderung einer ein Coronavirus umfassenden Virusinfektion in einem Subjekt, wobei die pharmazeutische Zusammensetzung ein rekombinantes humanes Surfactant-Protein D (rhSP-D) umfasst,
wobei die Virusinfektion SARS-CoV-2 umfasst und
wobei das SARS-CoV-2 ein S1-Protein umfasst, das eine N501Y-Mutation oder eine D614G-Mutation umfasst.

2. Zusammensetzung zur Verwendung nach Anspruch 1, wobei dem S1-Protein eine aus K417N und E484K ausgewählte Mutation fehlt.

3. Zusammensetzung zur Verwendung nach Anspruch 1 oder 2, wobei die pharmazeutische Zusammensetzung ferner einen Puffer, einen Zucker und ein Calciumsalz umfasst.

4. Zusammensetzung zur Verwendung nach Anspruch 3, wobei der Puffer ausgewählt ist aus der Gruppe bestehend aus Acetat, Citrat, Glutamat, Histidin, Succinat und Phosphat.

5. Zusammensetzung zur Verwendung nach Anspruch 3 oder 4, wobei der Puffer Histidin umfasst und wobei die Konzentration des Histidins 1 mM bis 10 mM beträgt.

6. Zusammensetzung zur Verwendung nach einem der Ansprüche 3 bis 5, wobei der Zucker ausgewählt ist aus der Gruppe bestehend aus Saccharose, Maltose, Lactose, Glucose, Fructose, Galactose, Mannose, Arabinose, Xylose, Ribose, Rhamnose, Trehalose, Sorbose, Melezitose, Raffinose, Thioglucose, Thiomannose, Thiofructose, Octa-O-acetyl-thiotrehalose, Thiosaccharose und Thiomaltose.

7. Zusammensetzung zur Verwendung nach einem der Ansprüche 3 bis 6, wobei der Zucker Lactose umfasst und wobei die Konzentration der Lactose 200 mM bis 300 mM beträgt.

8. Zusammensetzung zur Verwendung nach einem der Ansprüche 3 bis 7, wobei das Calciumsalz ausgewählt ist aus der Gruppe bestehend aus Calciumchlorid, Calciumbromid, Calciumacetat, Calciumsulfat und Calciumcitrat.

9. Zusammensetzung zur Verwendung nach einem der Ansprüche 3 bis 8, wobei das Calciumsalz Calciumchlorid umfasst und wobei die Konzentration des Calciumchlorids 1 mM bis 10 mM beträgt.

10. Zusammensetzung zur Verwendung nach einem der Ansprüche 3 bis 9, wobei die pharmazeutische Zusammensetzung einen pH-Wert von 5,0 bis 7,0 aufweist.

11. Zusammensetzung zur Verwendung nach einem der Ansprüche 3 bis 10, wobei die Konzentration des rhSP-D 0,1 mg/ml bis 10 mg/ml beträgt.

12. Zusammensetzung zur Verwendung nach einem der Ansprüche 1 bis 11, wobei das rhSP-D eine Aminosäuresequenz umfasst, die eine Identität von mindestens 95 % zu einer Aminosäuresequenz gemäß SEQ ID NO:02 aufweist.

13. Zusammensetzung zur Verwendung nach einem der Ansprüche 1 bis 12, wobei das Subjekt ein Säugetier ist.

14. Zusammensetzung zur Verwendung nach einem der Ansprüche 1 bis 13, wobei das S1-Protein eine N501Y-Mutation, eine D614G-Mutation und eine HV69-70-del-Mutation umfasst.

## Revendications

1. Composition pharmaceutique pour son utilisation dans le traitement ou l'amélioration d'une infection virale comprenant un coronavirus chez un sujet, dans laquelle la composition pharmaceutique comprend une protéine D du surfactant humain recombinante (rhSP-D),
dans laquelle l'infection virale comprend le SARS-CoV-2, et
dans laquelle le SARS-CoV-2 comprend une protéine S1 comprenant une mutation N501Y ou une mutation D614G.

2. Composition pour son utilisation selon la revendication 1, dans laquelle la protéine S1 est dépourvue de mutation choisie parmi K417N et E484K.

3. Composition pour son utilisation selon la revendication 1 ou 2, dans laquelle la composition pharmaceutique comprend en outre un tampon, un sucre et un sel de calcium.

4. Composition pour son utilisation selon la revendication 3, dans laquelle le tampon est choisi dans le groupe constitué par l'acétate, le citrate, le glutamate, l'histidine, le succinate et le phosphate.

5. Composition pour son utilisation selon la revendication 3 ou 4, dans laquelle le tampon comprend de l'histidine, et dans laquelle la concentration de l'histidine est de 1 mM à 10 mM.

6. Composition pour son utilisation selon l'une quelconque des revendications 3 à 5, dans laquelle le sucre est choisi dans le groupe constitué par le saccharose, le maltose, le lactose, le glucose, le fructose, le galactose, le mannose, l'arabinose, le xylose, le ribose, le rhamnose, le tréhalose, le sorbose, le mélézitose, le raffinose, le thioglucose, le thiomannose, le thiofructose, l'octa-O-acétyl-thiotréhalose, le thiosaccharose et le thiomaltose.

7. Composition pour son utilisation selon l'une quelconque des revendications 3 à 6, dans laquelle le sucre comprend du lactose, et dans laquelle la concentration du lactose est de 200 mM à 300 mM.

8. Composition pour son utilisation selon l'une quelconque des revendications 3 à 7, dans laquelle le sel de calcium est choisi dans le groupe constitué par le chlorure de calcium, le bromure de calcium, l'acétate de calcium, le sulfate de calcium et le citrate de calcium.

9. Composition pour son utilisation selon l'une quelconque des revendications 3 à 8, dans laquelle le sel de calcium comprend du chlorure de calcium, et dans laquelle la concentration du chlorure de calcium est de 1 mM à 10 mM.

10. Composition pour son utilisation selon l'une quelconque des revendications 3 à 9, dans laquelle la composition pharmaceutique a un pH de 5,0 à 7,0.

11. Composition pour son utilisation selon l'une quelconque des revendications 3 à 10, dans laquelle la concentration de la rhSP-D est de 0,1 mg/ml à 10 mg/ml.

12. Composition pour son utilisation selon l'une quelconque des revendications 1 à 11, dans laquelle la rhSP-D comprend une séquence d'acides aminés présentant au moins 95 % d'identité avec une séquence d'acides aminés de la SEQ ID NO:02.

13. Composition pour son utilisation selon l'une quelconque des revendications 1 à 12, dans laquelle le sujet est un mammifère.

14. Composition pour son utilisation selon l'une quelconque des revendications 1 à 13, dans laquelle la protéine S1 comprend une mutation N501Y, une mutation D614G et une mutation HV69-70 del.
